# EUROPEAN PATENT SPECIFICATION

(11) **EP 2 671 559 B1**
(45) Date of publication and mention of the grant of the patent: **27.04.2016**
(21) Application number: 12752930.3
(22) Date of filing: 01.03.2012
(51) Int. Cl.: A61H 1/02, A61H 3/00, A61B 5/11, A63B 22/02, A43B 3/00

(54) **AMBULATION TRAINING DEVICE AND AMBULATION TRAINING SYSTEM**
GEHTRAININGSVORRICHTUNG UND GEHTRAININGSSYSTEM
DISPOSITIF ET SYSTÈME D'ENTRAÎNEMENT À LA MARCHE

(30) Priority: 02.03.2011 JP 2011045318
(43) Date of publication of application: 11.12.2013
(73) Proprietor: University of Tsukuba, Tsukuba-shi, Ibaraki 305-8577 (JP); Cyberdyne Inc., Ibaraki 305-0818 (JP)
(72) Inventor: SANKAI Yoshiyuki, Tsukuba-shi Ibaraki 305-8577 (JP); HAYASHI Tomohiro, Tsukuba-shi Ibaraki 305-0818 (JP)
(74) Representative: Regimbeau
(86) International application number: PCT/JP2012/055223
(87) International publication number: WO 2012/118143

(56) References cited:
- WO-A1-2008/032967
- JP-A- 9 154 901
- JP-A- 2001 296 951
- JP-A- 2005 205 001
- JP-A- 2006 006 384
- JP-A- 2007 307 118
- JP-A- 2008 048 981
- JP-A- 2008 114 315
- JP-A- 2010 075 213
- JP-U- H0 615 658
- US-A1- 2010 004 577

## Description

### TECHNICAL FIELD

The present invention relates to a gait training device and system, and more particularly to a gait training device that operates in cooperation with a wearable motion assist device assisting a wearer to act or doing an action for the wearer, and a gait training system provided with the wearable motion assist device and the gait training device.

### BACKGROUND ART

Disabled persons and elderly persons often have difficulties to do an action that can easily be done by physically unimpaired persons. Therefore, various power assist devices have been developed in recent years to assist these persons to act or do an action for these persons.

As a power assist device, a wearable motion assist device (hereinafter merely referred to as a "motion assist device") fitted to a user (hereinafter referred to as a "wearer") has been known (see Patent Literature 1, for example). The motion assist device includes a muscle potential sensor (biosignal generating unit) that detects a muscle potential signal involved with a muscle activity of the wearer, a joint angle detecting unit that detects an angular movement of each joint of the wearer, a drive source, such as a drive motor, applying torque serving as assist power to the wearer, and a control unit that controls the drive source.

In the motion assist device, the control unit appropriately controls the drive motor based upon the detection result of the muscle potential sensor and the detection result of the joint angle detecting unit, whereby torque according to the wearer's will and suitable for the action of the wearer can be applied to the wearer. Thus, the motion assist device can assist a person to walk who has difficulty in walking. Such motion assist device can be used in cooperation with a gait training device as is for example disclosed in US 2010/0004577.

An existing gait training system externally applies power to lower extremities of a person having difficulty in walking to move the lower extremities, while supporting the body trunk, in order to make the person passively do a walking-like motion. However, this system does not facilitate the voluntary motion of the wearer. This type of system is based on a situation in which a patient has a certain level of muscle strength on his/her lower extremities. Therefore, it cannot be used for gait training for a patient having difficulty in active movement.

### CITATION LIST

### PATENT LITERATURE

Patent Literature 1: Japanese Unexamined Patent Publication No. 2005-230099

### SUMMARY

### TECHNICAL PROBLEM

The present invention aims to provide a gait training device that operates in cooperation with a wearable motion assist device assisting a wearer to act or doing an action for the wearer, and can realize a safe gait training even for a patient having difficulty in active movement of his/her lower extremities, and a gait training system provided with the wearable motion assist device and the gait training device.

### SOLUTION TO PROBLEM

According to one aspect of the present invention, there is provided a gait training device as disclosed in the appended claims. Preferably, the gait training device operates in cooperation with a wearable motion assist device specifying a walking phase of a wearer, and applying power to the wearer in correspondence to the walking phase of the wearer, the gait training device including a body weight support device that lifts up the wearer to support a weight applied on feet, a moving unit that is movable according to a walking motion of the wearer, and a control device that controls a moving speed of the moving unit and/or an amount of body-weight support by the body weight support device based upon a position of a center of gravity of the wearer detected by the wearable motion assist device.

According to one aspect of the present invention, in the gait training device, the control unit controls the moving speed of the moving unit and/or the amount of body-weight support by the body weight support device, when the position of the center of gravity of the wearer is outside a predetermined region.

According to one aspect of the present invention, in the gait training device, the control device acquires a walking speed of the wearer from the wearable motion assist device, and controls the moving speed of the moving unit based upon the walking speed.

According to one aspect of the present invention, in the gait training device, the body weight support device detects a tilt of a body of the wearer, and the control unit controls the moving speed of the moving unit and/or the amount of body-weight support by the body weight support device based upon the tilt of the body of the wearer detected by the body weight support device.

According to one aspect of the present invention, in the gait training device, the moving unit is a treadmill having a walking belt that rotates with a rotation of a roller, and the control unit controls a motor that rotates the roller.

According to one aspect of the present invention, in the gait training device, the moving unit has a wheel so as to be capable of moving with the body weight support device, and the control unit controls a motor that rotates the wheel.

According to one aspect of the present invention, in the gait training device, the wheel moves along a rail provided on a ceiling.

According to one aspect of the present invention, a gait training system includes a wearable motion assist device including a drive source that applies power to a wearer, a first detecting unit that detects a biopotential signal involved with a muscle activity of the wearer, a second detecting unit that detects an angle of a joint of the wearer, a third detecting unit that detects a sole load of the wearer, a first control unit that generates a first command signal for allowing the drive source to generate power according to the biopotential signal, a second control unit that specifies a walking phase of the wearer based upon the joint angle and the sole load, and generates a second command signal for allowing the drive source to generate power according to the phase, a synthesis unit that synthesizes the first command signal and the second command signal to generate a synthesis command signal, a generating unit that generates a drive current based upon the synthesis command signal, and supplies this drive current to the drive source, and a calculating unit that calculates a walking speed of the wearer, and the gait training device.

According to one aspect of the present invention, in the gait training system, the calculating unit acquires a length of stride of the wearer from a length of the leg of the wearer, and a transition of the joint angle of the wearer detected by the second detecting unit, and calculates the walking speed from the length of stride and a time from when the foot of the wearer is off the ground until it is put on the ground.

### ADVANTAGEOUS EFFECTS OF THE INVENTION

According to the present invention, even a patient having difficulty in active movement of his/her lower extremities can safely perform gait training.

### BRIEF DESCRIPTION OF THE DRAWINGS

FIG. 1 is a perspective view of an appearance of a gait training system according to an embodiment of the present invention.
FIG. 2 is a block diagram illustrating a configuration of the gait training system according to the embodiment.
FIG. 3 is a view illustrating an example of a task for classifying a motion of a wearer.
FIG. 4A is a view illustrating an example of shifting a wearer's weight during walking.
FIG. 4B is a view illustrating an example of shifting a wearer's weight during walking.
FIG. 5 is a perspective view of the wearer viewed from back.
FIG. 6 is a view illustrating a walking phase of the wearer.
FIG. 7 is a flowchart for describing a method of a gait training using the gait training system according to the embodiment.
FIG. 8 is a perspective view of an appearance of a gait training system according to a first modification.
FIG. 9 is a perspective view of an appearance of a gait training system according to a second modification.

### DESCRIPTION OF EMBODIMENTS

Embodiments of the present invention will be described with reference to the drawings.

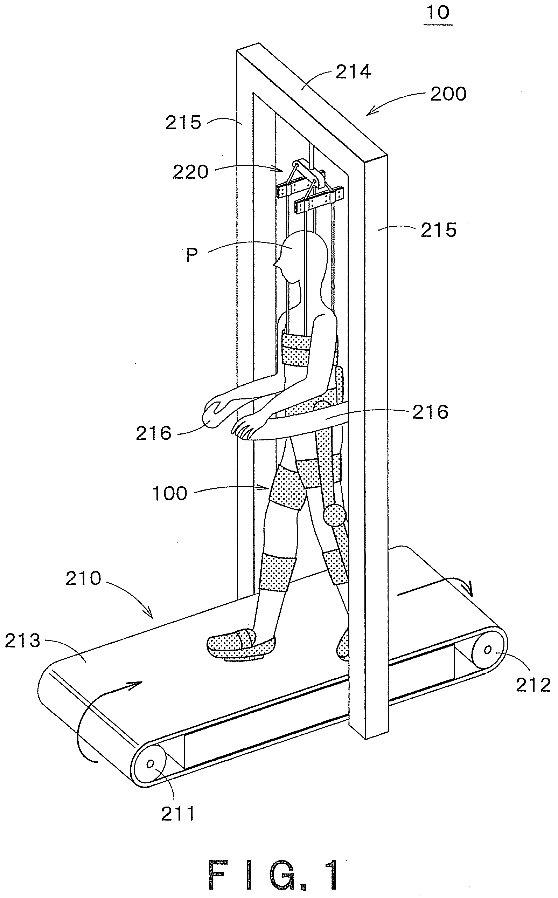

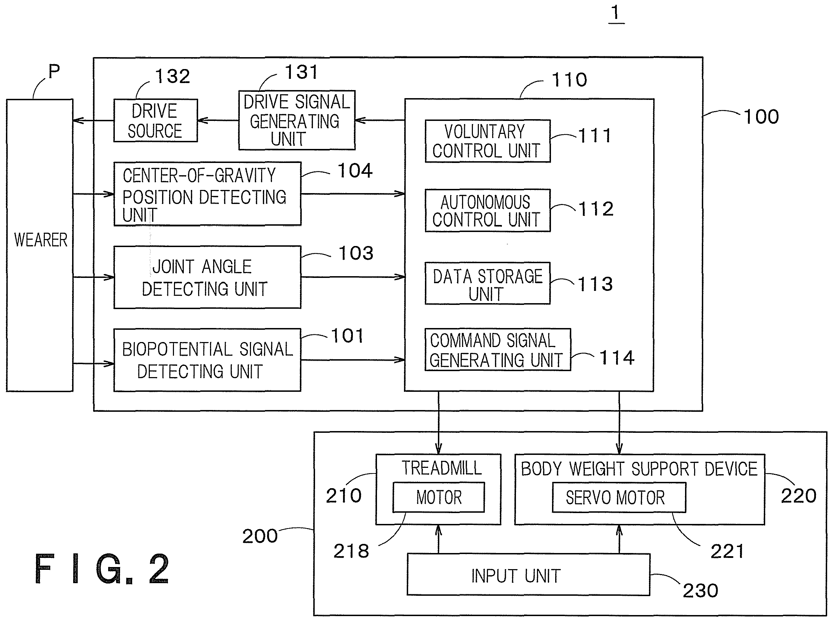

FIG. 1 is a perspective view of an appearance of a gait training system 1 according to an embodiment of the present invention. FIG. 2 is a block diagram illustrating a configuration of the gait training system 1. As illustrated in FIGS. 1 and 2, the gait training system 1 includes a wearable motion assist device 100 that assists a motion of a wearer P, and a gait training device 200 that helps the wearer P to do a gait training. The wearer P is a person who wears the wearable motion assist device 100. The wearable motion assist device 100 and the gait training device 200 are connected to each other wirelessly or by use of wires to enable intercommunication.

The gait training device 200 will firstly be described. The gait training device 200 includes a treadmill 210, and a body weight support device 220 that supports the weight (body weight) applied to the legs of the wearer P. The treadmill 210 has a walking belt 213 that rotates by the rotation of rollers 211 and 212. The walking belt 213 is arranged horizontally. A motor 218 (see FIG. 2) is connected to the roller 211 by a belt, and it can rotate the roller 211. The speed of the walking belt 213 can be changed by changing the revolution of the roller 211. The walking belt 213 may be arranged to be inclined in the longitudinal direction like a slope. When the slope is sharp, the load in the gait training can be increased, and when the slope is almost horizontal, the load can be adjusted to be small.

Two columns 215 whose top ends are connected by a frame 214 are provided on the right and left (on the near side and far side in FIG. 1) of the walking belt 213. An arm frame 216 is provided on the center of each column 215. The wearer P can hold the posture during the gait training by gripping the arm frames 216.

A load sensor may be mounted to the arm frame 216 in order to be capable of detecting how much weight is applied on the arm frame 216 by the wearer P during the gait training. A strain sensor (strain gauge) can be used for the load sensor, for example. The strain sensor detects the load from the strain when the load is applied to the arm frame 216. The shift of the weight of the wearer P during walking can correctly be detected by the load sensor mounted on the arm frame 216.

The body weight support device 220 has a lifting harness, for example. One end is connected to the frame 214, and the other end is connected to the wearer P or the wearable motion assist device 100. The body weight support device 220 lifts up the harness by a servo motor 221 (see FIG. 2). The amount of body-support weight in the weight applied to the legs of the wearer P is determined by how much the harness is lifted up by the servo motor 221. The larger the amount of lifting up the harness is, the larger the amount of body-weight support becomes, so that the weight applied on the legs of the wearer P decreases.

The lifting harness of the body weight support device 220 may be connected to both the wearer P and the wearable motion assist device 100.

The gait training device 200 includes an input unit 230 (see FIG. 2) that can adjust the revolution of the roller 211 by the motor 218 and the amount of body-weight support by the body weight support device 220. The wearer P can adjust the speed of the walking belt 213 or the weight applied on his/her foot by using this input unit 230. For example, the wearer P can set the speed of the walking belt 213 to be equal to (almost equal to) the walking speed of the wearer P by using the input unit 230. It may be configured such that the level of the speed of the walking belt 213 can be set higher or lower than the walking speed of the wearer P. The speed of the walking belt 213 may be set to be lower or higher than the current set speed.

The wearable motion assist device 100 will next be described with reference to FIG. 2. The wearable motion assist device 100 includes a biopotential signal detecting unit 101, a joint angle detecting unit 103, a center-of-gravity position detecting unit 104, a control device 110, a drive signal generating unit 131, and a drive source (actuator) 132.

The biopotential signal detecting unit 101 detects a muscle potential according to a muscle force generated from the wearer P. When a person intends to move, the will of moving is converted into an electric signal, and transmitted to a muscle from a brain through an internal nerve. In this case, the biopotential signal detecting unit 101 detects a biopotential signal generated on a surface of a skin.

The joint angle detecting unit 103 detects a joint angle according to the motion of the wearer P, and outputs the resultant to the control device 110.

The center-of-gravity position detecting unit 104 detects the position of the center of gravity according to the motion of the wearer P, and outputs the resultant to the control device 110.

The control device 110 includes a voluntary control unit 111, an autonomous control unit 112, a data storage unit 113, and a command signal synthesis unit 114.

The voluntary control unit 111 performs a filter process (smoothing process) and a signal process including an amplification to the biopotential signal (muscle potential signal) detected by the biopotential signal detecting unit 101. The voluntary control unit 111 generates a voluntary command signal for allowing the drive source (actuator) 132 to generate power according to the will of the wearer P by using the bipotential signal undergoing the signal process.

The data storage unit 113 stores a reference parameter database for specifying a phase of a task of the wearer P, and an assist parameter for assisting the motion of the wearer P according to the specified phase. The task is formed by classifying main motion patterns of a person. The phase is a series of minimum motion units forming each task.

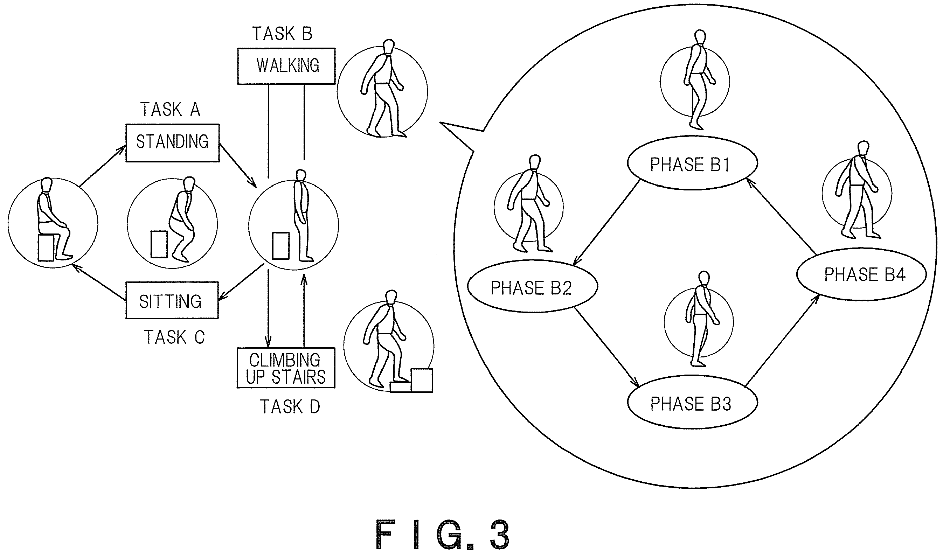

FIG. 3 illustrates one example of each task and each phase stored in the reference parameter database.

As illustrated in FIG. 3, the task formed by classifying the motion of the wearer P and stored in the reference parameter database includes a task A having standing-up motion data that indicates the change from a sitting position to a standing position, a task B having walking motion data that indicates the walking motion of the wearer P who stands up, a task C having sitting motion data that indicates the change from the standing position to the sitting position, and a task D having stairs climbing up/down data that indicates the motion of climbing up and climbing down the stairs from the standing position, for example.

Each task has plural phase data. For example, the task B for the walking motion has a phase B1 having motion data (joint angle, trajectory of the position of the center of gravity, variation in torque, change in the biopotential signal, etc.) when the wearer P steps forward with his/her right foot from the position where the wearer P stands with the his/her weight being put over his/her left foot, a phase B2 having motion data when the wearer P puts his/her right foot, which moves forward, on the ground, and shifts his/her weight, a phase B3 having motion data when the wearer P steps forward with his/her left foot from the position where the wearer P stands with his/her weight being put over his/her right foot, and a phase B4 having motion data when the wearer P puts his/her left foot, which is in front of the right foot, on the ground, and shifts his/her weight.

As a result of the analysis of general motions of a person, it is found that the typical motion patterns including the angle of each joint, and the shift of one's weight in each phase are fixed. Therefore, the typical joint angle and the shift of one's weight are experientially obtained for each phase forming a lot of basic motions (tasks) of a person, and they are stored in the reference parameter database.

Plural assist patterns are assigned to each phase, and a different assist is applied for each assist pattern even in the same phase.

For example, a person has a different walking pattern depending upon a size of a body, a muscle condition, and a walking speed. The walking pattern is also different depending upon the purpose of the motion (for example, rehabilitation purpose, training purpose, purpose of improving a gait, purpose of assisting (power of) a motion, etc). Even for the rehabilitation purpose, the appropriate motion patterns are different according to the degree of disability of a target person, and the progression of the rehabilitation.

Therefore, the assist suitable for the target motion pattern is also different. In order to select the optimum assist pattern from the plural assist patterns according to the assist suitable for the target motion pattern, many assist patterns are assigned to each phase.

The autonomous control unit 112 compares the parameter indicating the motion of the wearer, such as the joint angle detected by the joint angle detecting unit 103 and the position of the center of gravity detected by the center-of-gravity position detecting unit 104, and the reference parameter stored in the data storage unit 113, thereby specifying the task and phase of the motion of the wearer P. After specifying the phase according to the motion state of the wearer, the autonomous control unit 112 selects the optimum assist pattern, according to the purpose set beforehand, out of the assist patterns assigned to this phase, and generates an autonomous command signal for allowing the drive source (actuator) 132 to generate power according to this assist pattern.

The command signal synthesis unit 114 synthesizes the voluntary command signal generated by the voluntary control unit 111 and the autonomous command signal generated from the autonomous control unit 112, and outputs a synthesis command signal to the drive signal generating unit 131. The synthesis ratio of the voluntary command signal and the autonomous command signal may preliminarily be set for the phase of each task, and this ratio may be stored in the data storage unit 113.

The synthesis command signal has a waveform that allows the drive source 132 to generate power formed by combining power by the voluntary control, which changes from the start of the motion till the end of the motion, and power by the autonomous control for each phase.

The drive signal generating unit 131 generates a drive signal (drive current) according to the synthesis command signal, and feeds this signal to the drive source 132 to drive the drive source 132. The drive source 132 applies assist force (power) according to the drive signal to the wearer P.

A calculating unit (not illustrated) in the control device 110 obtains the length of stride from the leg length of the wearer P and the transition of the joint angle detected by the joint angle detecting unit 103, and calculates the walking speed from the length of stride and the time from when the foot is off the ground till the foot is put on the ground. The control device 110 can control the motor 218 in order that the walking belt 213 moves with the speed based upon the calculated walking speed.

The control device 110 can also control the amount of body-weight support by the body weight support device 220 based upon the position of the center of gravity detected by the center-of-gravity position detecting unit 104. For example, when the position of the center of gravity is outside a predetermined region, the control device 110 determines that the body of the wearer P greatly tilts. Therefore, the control device 110 controls the servo motor 221 to increase the amount of body-weight support by the body weight support device 220, thereby lifting up the wearer P. Thus, the posture of the wearer P can be stabilized. When the detection of the position of center of gravity by the center-of-gravity position detecting unit 104 is difficult, i.e., when an insole sensor of shoes 51 and 52 described later and a load sensor on a bottom of a stabilizer 53 are difficult to detect the load, the control device 110 determines that the wearer P is excessively lifted up, and controls the servo motor 221 to decrease the amount of body-weight support.

The control device 110 can control the speed of the walking belt 213 based upon the position of the center of gravity detected by the center-of-gravity position detecting unit 104. For example, when the position of the center of gravity is shifted forward from the predetermined region, the control device 110 determines the wearer P tilts forward, i.e., determines that the walking speed of the wearer P is higher than the speed of the walking belt 213. Therefore, the control device 110 increases the speed of the walking belt 213. On the contrary, when the position of the center of gravity is shifted rearward from the predetermined region, the control device 110 determines that the wearer P tilts backward, and decreases the speed of the walking belt 213.

When the position of the center of gravity shifted laterally outside the predetermined region, the control device 110 determines that the walking motion of the wearer P becomes unstable. Therefore, the control device 110 gradually decreases the speed of the walking belt 213, and gradually increases the amount of body-weight support, considering the safety of the wearer P.

The "predetermined region" here means the region where the position of the center of gravity can be changed during the walking motion. An ideal region experientially obtained by monitoring walking patterns of many persons can be set as the predetermined region, or the predetermined region can individually be set according to the attainment level of the wearer P for the walking motion.

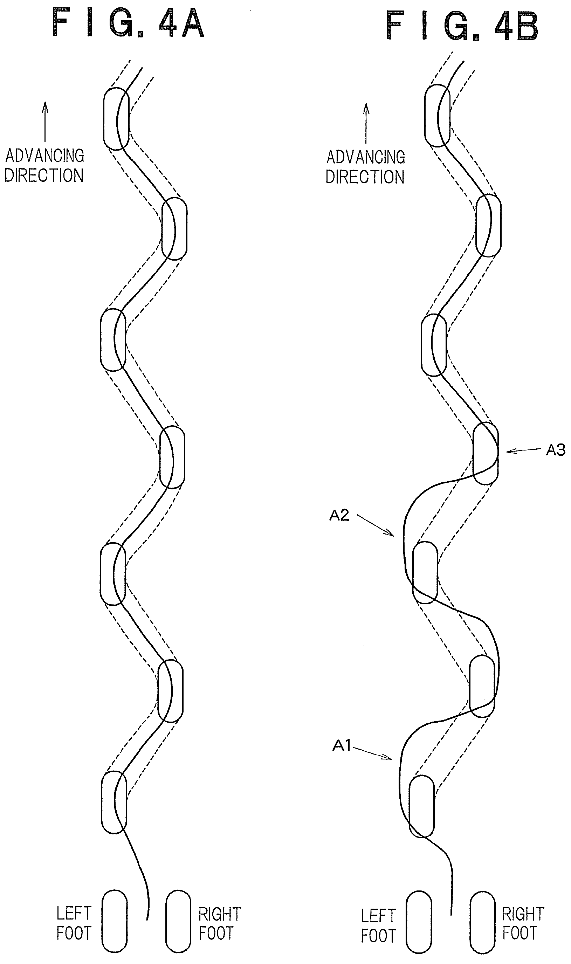

For example, the region where the position of the center of gravity is changed during the stable walking motion of the wearer P, i.e., the "predetermined region", is the region between two broken lines in FIG. 4A. When the position of the center of gravity (solid line) is present in this region, it can be determined that the wearer P stably walks.

FIG. 4B illustrates the example in which, when the position of the center of gravity is outside the "predetermined region", which means the walking motion of the wearer P is unstable, the amount of body-weight support by the body weight support device 220 and the speed of the walking belt 213 are controlled to stabilize the walking motion. When the center of gravity is shifted outside the foot as indicated by A1 in FIG. 4B, almost all load is applied on the stabilizer 53, so that it can be determined that the body is excessively shifted laterally. When the timing of the shift of the center of gravity is earlier as on a point A2, the body falls forward, so that it can be determined that the speed of the walking belt 213 is too slow. Since the control device 110 controls the amount of the body-weight support by the body weight support device 220 and the speed of the walking belt 213 on a point A3 based upon the determination described above, the subsequent walking motion of the wearer P can be stabilized.

When it is confirmed that the stable walking motion of the wearer P is continued (for example, the shift of the center of gravity is changed within the predetermined region during a predetermined period such as one minute or more or 20 or more steps), the control device 110 can control to gradually decrease the amount of body-weight support or gradually increase the speed of the walking belt 213 so as to gradually increase the load on the walking motion for the wearer P. In this way, the gait training can be done.

When the body weight support device 220 can detect the tilt of the body of the wearer P who is lifted up, the control device 110 may control the amount of body-weight support based upon the tilt of the body of the wearer P detected by the body weight support device 220. For example, a tilt detecting unit such as a gyro sensor may be mounted to the body trunk of the wearer P, and the control device 110 determines whether the body of the wearer P tilts or not based upon the detection signal.

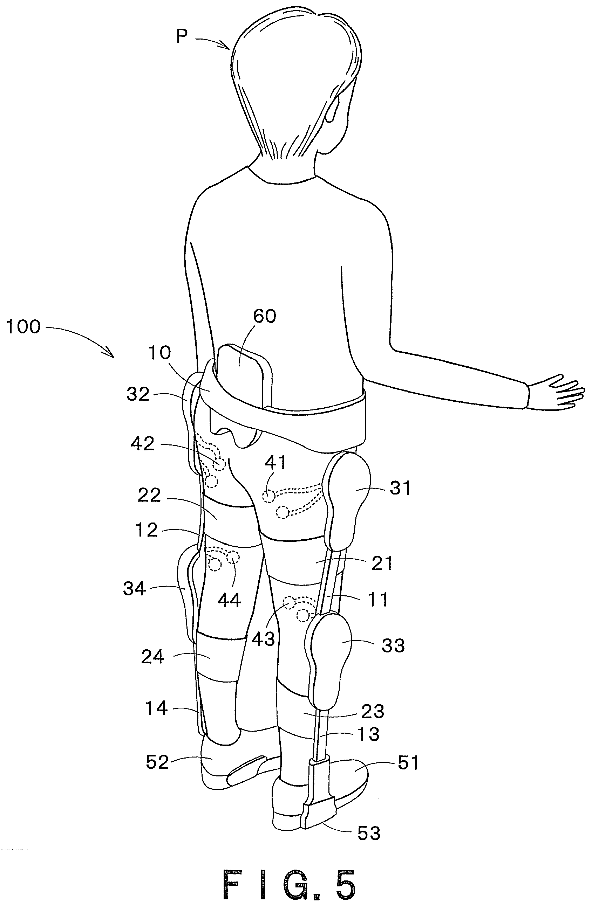

FIG. 5 is a perspective view of the wearer P viewed from the back. The wearable motion assist device 100 is a device for assisting the walking motion of a person having difficulty in active walking motion, such as a person with the impairment of lower limbs having difficulty in walking due to muscle loss of a skeletal muscle, or a patient doing rehabilitation for a walking motion. It detects a biosignal (surface muscle potential) generated upon generating muscle force by a signal from a brain, and operates to apply the driving force from the actuator to the wearer P based upon the detected biosignal.

When the wearer P wearing the wearable motion assist device 100 intends to walk on his/her will, drive torque needed for the walking motion is applied from the motion assist device 100 as assist power according to the generated biosignal. Therefore, even when the wearer P cannot walk because of the insufficient muscle force of his/her own, he/she can move his/her legs to walk by total force of the drive torque applied from the actuator according to the biosignal based upon his/her will.

In this case, the motion assist device 100 controls such that the assist power applied according to the shift of his/her weight involved with the walking motion reflects the will of the wearer P. Therefore, the actuator of the motion assist device 100 is controlled not to apply the load against the will of the wearer P, so that it does not interfere with the motion of the wearer P.

As illustrated in FIG. 5, the motion assist device 100 includes a waist frame 10, leg frames 11 to 14, fastening belts 21 to 24, power units 31 to 34, muscle potential sensors 41 to 44, shoes 51 and 52, and a control unit 60. The motion assist device 100 also includes a power supply (not illustrated) for feeding power to the power units 31 to 34 and the control unit 60. The power supply can be mounted on the waist frame 10.

The waist frame 10 is for supporting the waist of the wearer P, and is fixed to the trunk of the wearer P.

The power units 31 and 32 are connected to the waist frame 10 so as to be swingable with respect to the waist frame 10. The power units 31 and 32 are connected to the power units 33 and 34 via the leg frames 11 and 12. The power units 33 and 34 are connected to be swingable with respect to the leg frames 11 and 12.

The shoes 51 and 52 are connected to the power units 33 and 34 via the frames 13 and 14.

The power units 31 to 34 are mounted on the portions corresponding to joints on the thighs and on the lower legs (hip joints, and knee joints). The frames 11 and 12 are attached along the outside of the thigh of the wearer P, and the frames 13 and 14 are attached along the outside of the shin of the wearer P. Therefore, the frames 11 to 14 are configured to do the same motion as the legs of the wearer P.

The frames 11 and 12 are fastened to the thighs of the wearer P by the fastening belts 21 and 22. The frames 13 and 14 are fastened to the legs under the knees by the fastening belts 23 and 24.

The power units 31 to 34 include a drive motor. The rotation shaft of the drive motor transmits the drive torque to the frames 11 to 14, which are the driven members, through gears. The drive torque is transmitted to the legs of the wearer P through the fastening belts 21 to 24 as assist power.

The drive motor has an angular sensor detecting the joint angle. The angular sensor is configured by a rotary encoder that counts a number of pulses proportional to the joint angle, for example. The angular sensor outputs the detected joint angle to the control unit 60.

The power units 31 to 34 correspond to the joint angle detecting unit 103, the drive signal generating unit 131, and the drive source 132 in FIG. 2.

The muscle potential sensors 41 and 42 are attached to the hip of the wearer P for detecting the surface muscle potential of the muscle, such as gluteus maximus muscle, involved with the extension of the hip joint.

The muscle potential sensors 43 and 44 are attached on the back of the legs above the knees of the wearer P for detecting the surface potential of the muscle, such as biceps femoris muscle, involved with the extension of the knee joint.

Although not illustrated, a muscle potential sensor that is attached on the front of a groin of the wearer P for detecting the surface muscle potential of the muscle, such as rectus femoris muscle, involved with the flexion of the hip joint, and a muscle potential sensor that is attached on the front of the legs above the knees of the wearer P for detecting the surface muscle potential of the muscle, such as lateral great muscle or medial great muscle, involved with the extension of the knee joint are also provided.

The muscle potential sensor outputs the detected muscle potential to the control unit 60. The muscle potential sensors 41 to 44 correspond to the biopotential signal detecting unit 101.

The shoes 51 and 52 are provided with insole sensors (not illustrated). The insole sensor includes a reaction sensor detecting reaction force to the front side and back side of the right foot and the left foot, for example. The reaction sensor includes, for example, a piezoelectric element that outputs a voltage according to the applied load, and can detect the position of the center of gravity. The insole sensor outputs the detection result to the control unit 60.

The stabilizers 53 for preventing an inversion of the ankle joint are provided on the outside of the shoes 51 and 52. The stabilizer 53 has on its bottom the load sensor that detects the load applied on the ground (floor) through the frames 11 to 14 and outputs the resultant to the control unit 60. The load sensors and the insole sensors on the shoes 51 and 52 correspond to the center-of-gravity position detecting unit 104 in FIG. 2.

The insole sensors are provided in the shoes 51 and 52 like an insole. However, the similar sensors may be provided on the back (the surface in contact with the floor) of the shoe sole, not in the shoes 51 and 52. When the sensors are provided in the shoes, the load applied on the bottom of the foot of the wearer P, i.e., the position of the center of gravity of the wearer P alone, can be detected. On the other hand, when the sensor is provided on the back of the shoe sole, the load applied on the shoe sole, i.e., the position of the center of gravity of the total of the wearer P and the wearable motion assist device 100 can be detected. For example, when the wearer P is the severely disabled person, the weight is difficult to be applied on the bottom of the foot. Therefore, the position of the center of gravity can more precisely be detected by the measurement of the load applied on the shoe sole than by the measurement of the load on the insole. Accordingly, satisfactory gait training can be carried out.

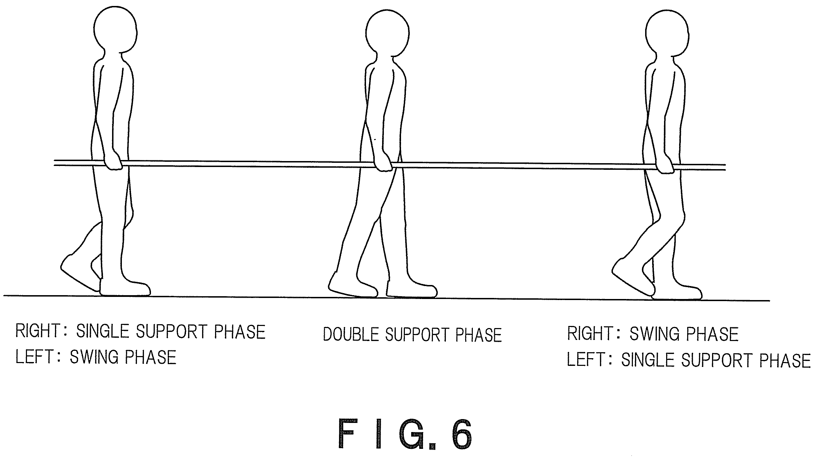

The walking phase of the wearer P receiving the gait training by utilizing the gait training system 1 includes three phases that are a single support phase, a double support phase, and a swing phase for each leg as illustrated in FIG. 6. The control of the actuator can be switched for each phase for each of the right leg and the left leg by the voluntary control unit 111 and the autonomous control unit 112.

Specifically, the voluntary control unit 111 determines whether the wearer P swings his/her leg based upon whether the biopotential detected by the biopotential signal detecting unit 101 exceeds a threshold value. The autonomous control unit 112 calculates the sole load ratio of the right foot and the left foot by using the load on the bottom of the foot detected by the center-of-gravity position detecting unit 104, and determines which one of the left single support phase, the double support phase, and the right single support phase the sole load ratio indicate.

When the biopotential of the right leg exceeds the threshold value, and the sole load ratio indicates the left single support phase, the control device 110 starts the assist for the swing phase for the right leg, and starts the assist for the single support phase for the left leg. On the contrary, when the biopotential of the left leg exceeds the threshold value, and the sole load ratio indicates the right single support phase, the control device 110 starts the assist for the swing phase for the left leg, and starts the assist for the single support phase for the right leg. Alternatively, when the sole load ratio indicates the double support phase, the control device 110 performs the assist for keeping the double support.

The assist process in each walking phase will next be described.

In the double support phase during walking, a person bears his/her weight on both feet, and shifts the weight on the front foot in preparation for the swing of the idling leg. In the present embodiment, the upper body is supported by the body weight support device 220 and the arm frame 216, so that the control device 110 applies torque to the power units 33 and 34 on the knee joints in order to prevent giving-way phenomenon. The control device 111 makes a feedback control in order that the hip joint is in an erect standing position. The feedback control made for the back leg in this case interferes with the motion of the wearer P to shift the weight on the front leg. Therefore, the feedback control is made only for the front leg. The target angle and the target angular speed of the angular sensors of the power units 31 and 32 on the hip joint are both zero.

In the single support phase, the wearer P bears his/her weight on one leg, and makes a motion of advancing the body trunk. The knee joints have a function of supporting the weight as in the double support phase, so that the control same as the control for the double support phase is applied. On the other hand, the hip joints have to be extended to advance the body trunk. Therefore, the control device 110 performs a trajectory tracking control aiming an angle pattern of the extension of the hip joint during walking by an able-bodied person.

In the swing phase, the wearer makes a series of motions in which the knee and the hip joint are bent in the first half, and then, they extend in the latter half to swing the leg forward to prepare for the next landing. In the present embodiment, the control device 110 performs the trajectory tracking control aiming the angle and the trajectory pattern of the angular sped of the knee and the hip joint of an able-bodied person during the swing phase.

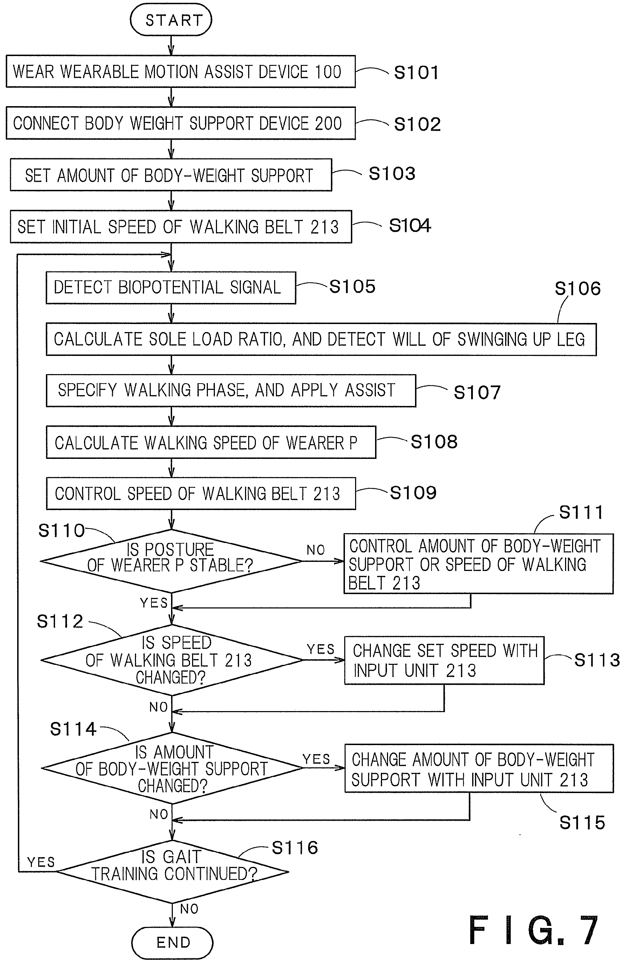

Subsequently, the method of the gait training utilizing the gait training system 1 will be described with reference to the flowchart in FIG. 7.
(Step S101) The wearer wears the wearable motion assist device 100.
(Step S102) The wearer P gets on the walking belt 213 of the treadmill 210, and is coupled to the body weight support device 220. The wearable motion assist device 100 may preliminarily be coupled to the body weight support device 220, and with this state, a person may wear the wearable motion assist device 100, in other words, the person may get on to the wearable motion assist device 100 coupled to the body weight support device 220.
(Step S103) The wearer P or an operator sets the amount of body-weight support by using the input unit 230. For example, the amount of body-weight support of 50% is set, whereby the load applied to the feet of the wearer P is reduced.
(Step S104) The wearer P or the operator sets an initial speed of the walking belt 213 by using the input unit 230. For example, 0.2 km/h is set.
(Step S105) The biopotential signal generating unit 101 detects the biopotential signal of the wearer P. The center-of-gravity position detecting unit 104 detects the left and right sole loads of the wearer P.
(Step S106) The control device 110 calculates the sole load ratio from the left and right sole loads, and determines which one of the left single support phase, the double support phase, and the right single support phase the wearer P has. The control device 110 also determines whether the wearer P intends to swing his/her leg based upon whether the biopotential exceeds the threshold value.
(Step S107) The control device 110 specifies the walking phase of each leg based upon the determination result in step S107, and applies the assist corresponding to the specified walking phase. Thus, the wearer P can walk.
(Step S108) The control device 110 calculates the walking speed of the wearer P. Specifically, the control device 110 firstly obtains the length of stride from the leg length of the wearer P and the transition of the joint angle detected by the joint angle detecting unit 103, and calculates the walking speed from the obtained length of stride and the time from when the foot is off the ground till the foot is put on the ground.
(Step S109) The control device 110 decides the speed of the walking belt 213 based upon the walking speed calculated in step S108, and controls the motor 218. For example, the control device 110 controls the motor 218 in order that the walking speed of the wearer P and the speed of the walking belt 213 become equal to each other.
(Step S110) The control device 110 determines whether the posture of the wearer P is stable or not. When determining that the posture is unstable, the control device 110 proceeds to step S111. When determining that the posture is stable, the control device 110 proceeds to step S112. For example, when the position of the center of gravity detected by the center-of-gravity position detecting unit 104 is outside the predetermined region, the control device 110 determines that the posture of the wearer P is unstable.
(Step S111) The control device 110 controls the amount of body-weight support by the body weight support device 220 in order to stabilize the posture of the wearer P. For example, the control device 110 increases the amount of body-weight support to further lift up the wearer P. The control device 110 may decrease the speed of the walking belt 213.
(Step S112) When changing the speed of the walking belt 213, the control device proceeds to step S113, and when the control device 110 does not change the speed, it proceeds to step S114.
(Step S113) The set speed of the walking belt 213 is changed by using the input unit 230. For example, how much the speed of the walking belt 213 increases more than the walking speed is inputted to the input unit 230. Thereafter, in step S109, the sped of the walking belt 213 is decided based upon this setting, and the motor 218 is controlled.
(Step S114) When changing the amount of body-weight support by the body weight support device 220, the control device 110 proceeds to step S115, and when it does not change the amount, it proceeds to step S116.
(Step S115) The setting of the amount of body-weight support is changed by using the input unit 230. Thus, the load applied on the feet of the wearer P is adjusted, whereby the wearer P can hold the desired posture.
(Step S116) When the gait training is continued, the process returns to step S105.

As described above, according to the gait training system according to the present embodiment, the wearer P is lifted up by the body weight support device 220 to stabilize the posture, and the wearable motion assist device 100 applies assist power to the wearer P to assist the walking motion. The control device 110 of the wearable motion assist device 100 can control the speed of the walking belt 213 of the treadmill 210 based upon the walking speed of the wearer P, and can control the amount of body-weight support by the body weight support device 220 based upon the position of the center of gravity and the tilt of the body of the wearer P. Accordingly, even a patient having difficulty in active movement of his/her lower extremities can safely carry out the gait training without worrying about a risk of fall.

In the embodiment described above, it is determined whether the posture of the wearer P is stable or not based upon the position of the center of gravity detected by the center-of-gravity position detecting unit 104 and the tilt of the body of the wearer P detected by the body weight support device 220. However, a load sensor may be mounted to the arm frame 216, and it may be determined that the posture of the wearer P is unstable, when the load detected by the load sensor becomes not less than a predetermined value. This is based upon the situation in which, when the posture becomes unstable, the wearer P tries to stabilize the posture by placing the weight on the arm frame 216.

In the embodiment described above, as for the assist for the knee joint, the muscle potential of the muscle, such as biceps femoris muscle, lateral great muscle, and medial great muscle, involved with the fixation and extension of the corresponding joint is detected. However, the potential of the abdominal muscle may be detected to sense the wearer's will of swinging up the leg. With this process, the will of swinging up the leg can be sensed even if the voluntary biopotential signal is difficult to be detected from the vicinity of the major hip flexors such as rectus femoris muscle.

In the embodiment described above, the control device 110 calculates the walking speed of the wearer P (step S108), and controls the motor 218 in order that the speed of the walking belt 213 becomes the speed corresponding to the calculated walking speed (step S109). However, this process may be skipped. In this case, the control device 110 controls the amount of body-weight support and/or the speed of the walking belt 213 based upon the postural stability (the position of the center of gravity) of the wearer P or the instruction of change via the input unit 230.

### (First modification of gait training device 200)

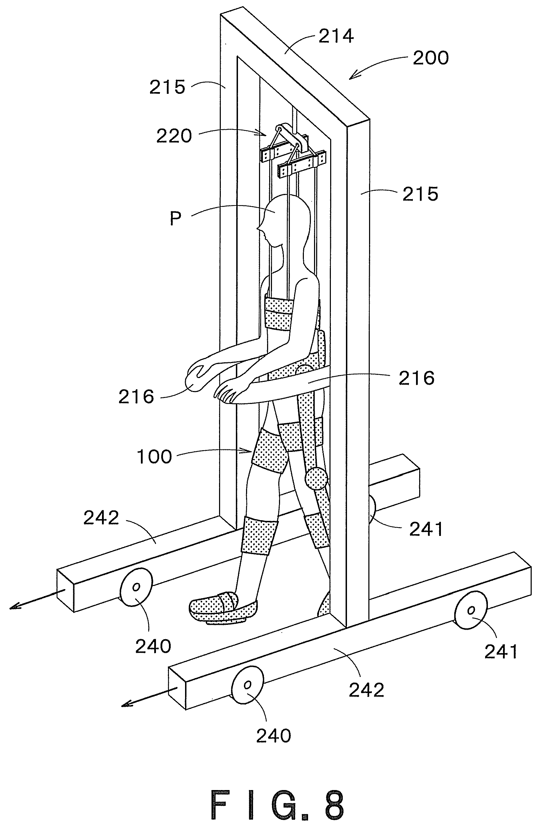

The gait training device 200 according to the embodiment described above is a floor-standing type using the treadmill 210. However, as illustrated in FIG. 8, the gait training device may be a self-propelled device provided with a pair of left and right front wheels 240 and a pair of left and right rear wheels 241. The front wheels 240 and the rear wheels 241 are coupled by the frame 242, and the column 215 is vertically provided to the frame 242.

The motor 218 (see FIG. 2) drives the front wheels 240 and the rear wheels 241, whereby the gait training device 200 can move longitudinally with the speed according to the walking speed of the wearer P. The control device 110 can control the moving speed of the self-propelled gait training device 200, like the control for the walking belt 213 in the embodiment described above.

According to this configuration, the wearer P can do the gait training exercise, while actually walking on the floor.

The load sensor is mounted on the arm frame 216, and the front wheels 240 and the rear wheels 241 are driven (rotated) according to the balance of the detected load, whereby the moving direction of the gait training device 200 can be changed to the right or to the left.

The device illustrated in FIG. 8 has four wheels. However, the device may have three wheels, or five or more wheels. The motor 218 drives at least one wheel in order that the gait training device 200 can move in the longitudinal direction.

### (Second modification of gait training device 200)

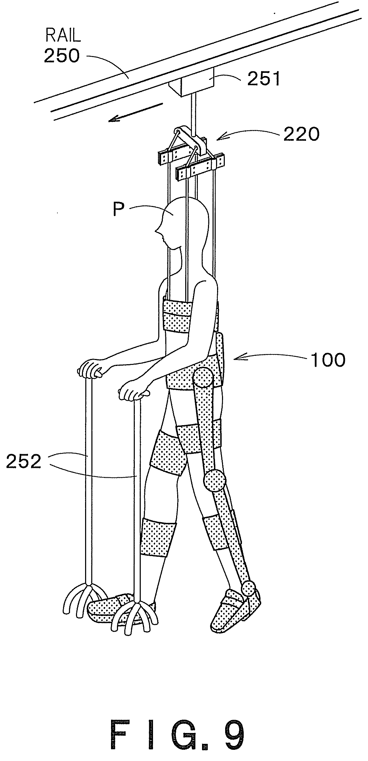

As illustrated in FIG. 9, the body weight support device 220 may move along a rail 250 provided on a ceiling. One end of the lifting harness is connected to a drive unit 251. The drive unit 251 is provided with the servo motor 221 (see FIG. 2) for lifting up the harness, a wheel (not illustrated) moving along the rail 250, and the motor 218 (see FIG. 2) for rotating the wheel. The drive unit 251 moves with the speed according to the walking speed of the wearer P.

Even with this configuration, the wearer P can do the gait training exercise, while actually walking on the floor.

A load sensor may be mounted on a stick 252 in order that the wearer P can find how much weight the wearer P applies on the stick 252 during walking. A strain sensor (strain gauge) can be used for the load sensor, for example. The strain sensor obtains the load from the strain when the load is applied on the stick 252. Since the load sensor described above is mounted on the stick 252, the shift of the weight of the wearer P during walking can correctly be detected.

In the embodiment and modifications described above, the control device 110 of the wearable motion assist device 100 controls the motor 218 or the servo motor 221. However, a control unit that controls the motor 218 based upon the walking speed of the wearer P or controls the servo motor 221 according to the posture of the wearer P may be provided to the gait training device 200. In this case, the control unit acquires information about the walking speed or posture (position of the center of gravity) of the wearer P from the wearable motion assist device 100, and controls the motor 218 and the servo motor 221.

At least a part of the gait training system 1 described in the embodiment may be realized by hardware or software. When it is realized by software, a program realizing at least some functions of the gait training system may be stored on a recording medium such as a flexible disk or CD-ROM, and may allow a computer to read and execute this program. The recording medium is not limited to a removable medium such as a magnetic disk or optical disk, but may be non-removable recording medium such as a hard disk drive or a memory.

A program realizing at least some functions of the gait training system 1 may be distributed through a communication line (including wireless communication) such as the Internet. The program may also be encrypted, modulated, or compressed, and with this state, may be stored on a recording medium through a wired communication or wireless communication such as the Internet for distribution.

In the wearable motion assist device 100 in the embodiment described above, the fastening belts 21 to 24 are directly fastened to the thighs or the legs under the knees of the wearer P. However, in order to fix the frames 11 to 14 on the legs of the wearer P, curved cuffs may be provided on the frames 11 to 14, and the fastening belts 21 to 24 may be wrapped after the cuffs are put on a posterior half of the thighs and the legs under the knees. When the cuff descried above is used, the power of the actuator can efficiently be transmitted to the legs of the wearer P.

The cuff may be put on the back of the thigh or the leg under the knee. The cuff may be put on the thigh and the leg under the knee from the inverse direction. During the normal walking motion, the wearer moves with his/her leg swinging forward. When the cuff is put from the back of the thigh or the leg under the knee, the power of the actuator can efficiently be transmitted to the legs. However, when the wearer P is severely disabled, it is preferable that the cuff is put on the thigh from the back, and the cuff is put on the leg under the knee from the front. The severely disabled person has difficulty in concentrating his/her energy in the feet, and hence, his/her knees are easy to be in flexion. Therefore, the cuff put on the lower thigh from the front can more effectively support the weight to easily assist the walking motion than the cuff put on the lower thigh from the back.

The present invention is not limited to the embodiments described above, and can be embodied by modifying the constituents without departing from the scope of the invention. Various inventions can be formed by an appropriate combination of the multiple constituents described in the embodiments. For example, some constituents may be eliminated from all constituents described in the embodiments. The constituents in different embodiments may appropriately be combined.

### REFERENCE SIGNS LIST

- 1: GAIT TRAINING SYSTEM
- 100: WEARABLE MOTION ASSIST DEVICE
- 101: BIOPOTENTIAL SIGNAL DETECTING UNIT
- 103: JOINT ANGLE DETECTING UNIT
- 104: CENTER-OF-GRAVITY POSITION DETECTING UNIT
- 110: CONTROL DEVICE
- 131: DRIVE SIGNAL GENERATING UNIT
- 132: DRIVE SOURCE (ACTUATOR)
- 200: GAIT TRAINING DEVICE
- 210: TREADMILL
- 220: BODY WEIGHT SUPPORT DEVICE

## Claims

1. A gait training device (200) for a wearer (P), intended to operate in cooperation with a wearable motion assist device (100) for detection of a position of a center of gravity of the wearer (P) comprising:
a body weight support device (220) that lifts up the wearer (P) to support a weight applied on feet;
a moving unit that is movable according to a walking motion of the wearer (P); and
**characterized by** a control device (110) that controls a moving speed of the moving unit and/or an amount of body-weight support by the body weight support device (220) based upon the position of the center of gravity of the wearer (P) detected by the wearable motion assist device (100).

2. The gait training device (200) according to claim 1, wherein
the control device (110) controls the moving speed of the moving unit and/or the amount of body-weight support by the body weight support device (220), when the position of the center of gravity of the wearer (P) is outside a predetermined region.

3. The gait training device (200) according to claim 1 or 2, wherein
the control device (110) acquires a walking speed of the wearer (P) from the wearable motion assist device (100), and controls the moving speed of the moving unit based upon the walking speed.

4. The gait training device (200) according to any one of claims 1 to 3, wherein
the body weight support device (220) detects a tilt of a body of the wearer (P), and
the control device (110) controls the moving speed of the moving unit and/or the amount of body-weight support by the body weight support device (220) based upon the tilt of the body of the wearer (P) detected by the body weight support device (220).

5. The gait training device (200) according to any one of claims 1 to 4, wherein
the moving unit is a treadmill (210) having a walking belt (213) that rotates with a rotation of a roller (211), and
the control device (110) controls a motor (218) that rotates the roller (211).

6. The gait training device (200) according to any one of claims 1 to 4, wherein
the moving unit has a wheel (240) so as to be capable of moving with the body weight support device, and
the control device (110) controls a motor that rotates the wheel.

7. The gait training device (200) according to claim 6, wherein
the wheel moves along a rail (250) provided on a ceiling.

8. A gait training system (1) comprising:
the gait training device (200) according to any one of claims 1 to 7; and
the wearable motion assist device (100) including:
a drive source (132) that applies power to the wearer (P);
a first detecting unit (101) that detects a biopotential signal involved with a muscle activity of the wearer (P);
a second detecting unit (103) that detects an angle of a joint of the wearer (P);
a third detecting unit (104) that detects a sole load of the wearer (P);
a first control unit (111) that generates a first command signal for allowing the drive source (132) to generate power according to the biopotential signal;
a second control unit (112) that specifies a walking phase of the wearer (P) based upon the joint angle and the sole load, and generates a second command signal for allowing the drive source (132) to generate power according to the phase;
a synthesis unit (114) that synthesizes the first command signal and the second command signal to generate a synthesis command signal;
a generating unit (131) that generates a drive current based upon the synthesis command signal, and supplies this drive current to the drive source (132); and
a calculating unit that calculates a walking speed of the wearer (P).

9. The gait training system (1) according to claim 8, wherein
the calculating unit acquires a length of stride of the wearer (P) from a length of the leg of the wearer (P), and a transition of the joint angle of the wearer (P) detected by the second detecting unit (103), and calculates the walking speed from the length of stride and a time from when the foot of the wearer (P) is off the ground until it is put on the ground.

10. The gait training system (1) according to claim 8 or 9, wherein
the wearable motion assist device (100) includes:
a first frame (11) and a second frame (13), the first frame (11) and the second frame (13) being attached along the leg of the wearer (P) to be capable of being bent;
a first cuff (21) mounted on the first frame (11) and put on a thigh of the wearer (P); and
a second cuff (23) mounted on the second frame (13) and put on the leg under the knee of the wearer (P).

11. The gait training system (1) according to claim 10, wherein
the first cuff (21) is put on the back of the thigh of the wearer (P), and the second cuff (23) is put on the front of the leg under the knee of the wearer (P).

## Patentansprüche

1. Gehtrainings-Vorrichtung (200) für einen Getragenen (P), der beabsichtigt in Zusammenarbeit mit einer tragbaren Bewegungs-Hilfsvorrichtung (100) zu arbeiten, um die Position einer Schwerpunktmitte des Getragenen (P) zu detektieren, umfassend:
eine Körpergewichts-Stützungsvorrichtung (220), die den Getragenen (P) aufrichtet, um das auf den Füßen lastende Gewicht abzustützen;
eine Bewegungseinheit, die entsprechend einer Gehbewegung des Getragenen (P) bewegbar ist; und
**gekennzeichnet durch** eine Steuerungsvorrichtung (110), die eine Bewegungsgeschwindigkeit der Bewegungseinheit und/oder einen Betrag der Körpergewichts-Stützung **durch** die Körpergewichts-Stützungsvorrichtung (220) abhängig von der Position der Schwerpunktmitte des Getragenen (P), die von der Bewegungs-Hilfsvorrichtung (100) detektiert wird, steuert.

2. Gehtrainings-Vorrichtung (200) nach Anspruch 1, wobei die Steuerungsvorrichtung (110) die Bewegungsgeschwindigkeit der Bewegungseinheit und/oder den Betrag der Körpergewichts-Stützung durch die Körpergewichts-Stützungsvorrichtung (220) steuert, wenn sich die Position der Schwerpunktmitte des Getragenen (P) außerhalb eines vorbestimmten Bereichs befindet.

3. Gehtrainings-Vorrichtung (200) nach Anspruch 1 oder 2, wobei die Steuerungsvorrichtung (110) eine Gehgeschwindigkeit des Getragenen (P) von der tragbaren Bewegungs-Hilfsvorrichtung (100) ermittelt, und die Bewegungsgeschwindigkeit der Bewegungseinheit abhängig von der Gehgeschwindigkeit steuert.

4. Gehtrainings-Vorrichtung (200) nach einem der Ansprüche 1 bis 3, wobei
die Körpergewichts-Stützungsvorrichtung (220) eine Neigung des Körpers des Getragenen (P) detektiert, und wobei
die Steuerungsvorrichtung (110) die Bewegungsgeschwindigkeit der Bewegungseinheit und/oder den Betrag der Körpergewichts-Stützung der Körpergewichts-Stützungsvorrichtung (220) abhängig von der Neigung des Körpers des Getragenen (P), der von der Körpergewichts-Stützungsvorrichtung (220) detektiert wird, steuert.

5. Gehtrainings-Vorrichtung (200) nach einem der Ansprüche 1 bis 4, wobei die Bewegungseinheit ein Laufbandgerät (210) ist, das ein Laufband (213) aufweist, das mit der Drehung einer Walze (211) umläuft, und wobei
die Steuerungsvorrichtung (110) einen Motor (218) steuert, der die Walze (211) zum Drehen antreibt.

6. Gehtrainings-Vorrichtung (200) nach einem der Ansprüche 1 bis 4, wobei
die Bewegungseinheit ein Rad (240) aufweist, um somit beschaffen zu sein, sich mit der Körpergewichts-Stützungsvorrichtung zu bewegen, und wobei
die Steuerungsvorrichtung (110) einen Motor steuert, der das Rad zum Drehen antreibt.

7. Gehtrainings-Vorrichtung (200) nach Anspruch 6, wobei das Rad sich entlang einer Schiene (250) bewegt, die an einer Raumdecke angebracht ist.

8. Gehtrainings-System umfassend:
die Gehtrainings-Vorrichtung (200) nach einem der Ansprüche 1 bis 7; und
die tragbare Bewegungs-Unterstützungsvorrichtung (100) aufweisend:
eine Antriebsquelle (132), die Kraft auf den Getragenen (P) anwendet;
eine erste Detektionseinheit (101), die ein Biopotential-Signal detektiert, das mit der Muskelaktivität des Getragenen (P) einhergeht;
eine zweite Detektionseinheit (102), die den Winkel eines Gelenks des Getragenen (P) detektiert;
eine dritte Detektionseinheit (103), die eine Schuhsohlen-Belastung des Getragenen (P) detektiert;
eine erste Steuerungseinheit (111), die ein erstes Befehlssignal erzeugt, um es der Antriebsquelle (132) zu ermöglichen, Leistung entsprechend dem Biopotential-Signal zu erzeugen;
eine zweite Steuerungseinheit (112), die eine Gehphase des Getragenen (P) abhängig von dem Gelenk-Winkel und der Schuhsohlen-Belastung spezifiziert, und die ein zweites Befehlssignal erzeugt, um es der Antriebsquelle (132) zu ermöglichen, Kraft entsprechend der Phase zu erzeugen;
eine Syntheseeinheit (114), die das erste Befehlssignal und das zweite Befehlssignal synthetisiert, um ein Synthese-Befehlssignal zu erzeugen;
eine Erzeugungs-Einheit (131), die einen Antriebsstrom abhängig von dem Synthese-Befehlssignal erzeugt, und die diesen Antriebsstrom für die Antriebsquelle (132) bereitstellt; und
eine Recheneinheit, die eine Gehgeschwindigkeit des Getragenen (P) berechnet.

9. Gehtrainings-System (1) nach Anspruch 8, wobei die Recheneinheit eine Schrittlänge des Getragenen (P) von einer Beinlänge des Getragenen (P) und von einem Übergang des von der zweiten Detektionseinheit (103) detektierten Gelenkwinkels des Getragener (P) ermittelt, und die die Gehgeschwindigkeit von der Schrittlänge und einer Zeit von dem Moment an berechnet, wenn der Fuß des Getragenen (P) vom Untergrund abgehoben ist, bis zu dem Moment, an dem er wieder auf den Untergrund gesetzt wird.

10. Gehtrainings-System (1) nach Anspruch 8 oder 9, wobei die tragbare Bewegungs-Unterstützungsvorrichtung (100) aufweist:
einen erstes Gestell (11) und ein zweites Gestell (13), wobei das erste Gestell (11) und das zweite Gestell (13) entlang der Beine des Getragenen (P) angeordnet sind, um beschaffen zu sein, abgewinkelt zu werden;
eine erste Manschette (21), die an dem ersten Gestell (11) montiert ist und auf einem Oberschenkel des Getragenen (P) angebracht ist;
eine zweite Manschette (23), die an dem zweiten Gestell (13) montiert ist und auf dem Bein unterhalb des Knies des Getragenen (P) angebracht ist.

11. Gehtrainings-System (1) nach Anspruch 10, wobei die erste Manschette (21) auf der Rückseite des Oberschenkels des Getragenen (P) angebracht wird, und wobei die zweite Manschette (23) auf der Vorderseite des Beines unterhalb des Knies des Getragenen (P) angebracht wird.

## Revendications

1. Dispositif d'apprentissage de la marche (200) pour un utilisateur (P), destiné à fonctionner en coopération avec un dispositif d'assistance au mouvement pouvant être porté (100) pour la détection d'une position d'un centre de gravité de l'utilisateur (P) comprenant :
un dispositif de support de poids de corps (220) qui soulève l'utilisateur (P) pour supporter un poids appliqué sur les pieds ;
une unité mobile qui peut être déplacée conformément à un mouvement de marche de l'utilisateur (P) ; et
**caractérisé par** un dispositif de commande (110) qui commande une vitesse de déplacement de l'unité mobile et/ou une quantité de support de poids de corps par le dispositif de support de poids de corps (220) sur la base de la position du centre de gravité de l'utilisateur (P) détectée par le dispositif d'assistance au mouvement pouvant être porté (100).

2. Dispositif d'apprentissage de la marche (200) selon la revendication 1, dans lequel
le dispositif de commande (110) commande la vitesse de déplacement de l'unité mobile et/ou la quantité de support de poids de corps par le dispositif de support de poids de corps (220), lorsque la position du centre de gravité de l'utilisateur (P) est à l'extérieur d'une région prédéterminée.

3. Dispositif d'apprentissage de la marche (200) selon la revendication 1 ou 2, dans lequel
le dispositif de commande (110) acquiert une vitesse de marche de l'utilisateur (P) à partir du dispositif d'assistance au mouvement pouvant être porté (100), et commande la vitesse de déplacement de l'unité mobile sur la base de la vitesse de marche.

4. Dispositif d'apprentissage de la marche (200) selon l'une quelconque des revendications 1 à 3, dans lequel
le dispositif de support de poids de corps (220) détecte une inclinaison du corps de l'utilisateur (P), et
le dispositif de commande (110) commande la vitesse de déplacement de l'unité mobile et/ou la quantité de support de poids de corps par le dispositif de support de poids de corps (220) sur la base de l'inclinaison du corps de l'utilisateur (P) détectée par le dispositif de support de poids de corps (220).

5. Dispositif d'apprentissage de la marche (200) selon l'une quelconque des revendications 1 à 4, dans lequel
l'unité mobile est un tapis roulant (210) comportant une courroie de marche (213) qui tourne avec la rotation d'un rouleau (211), et
le dispositif de commande (110) commande un moteur (218) qui fait tourner le rouleau (211).

6. Dispositif d'apprentissage de la marche (200) selon l'une quelconque des revendications 1 à 4, dans lequel
l'unité mobile comporte une roue (240) de manière à pouvoir se déplacer avec le dispositif de support de poids de corps, et
le dispositif de commande (110) commande un moteur qui fait tourner la roue.

7. Dispositif d'apprentissage de la marche (200) selon la revendication 6, dans lequel
la roue se déplace le long d'un rail (250) prévu sur un plafond.

8. Système d'apprentissage de la marche (1) comprenant :
le dispositif d'apprentissage de la marche (200) selon l'une quelconque des revendications 1 à 7 ; et
le dispositif d'assistance au mouvement pouvant être porté (100) comprenant :
une source d'entraînement (132) qui applique une puissance à l'utilisateur (P) ;
une première unité de détection (101) qui détecte un signal de biopotentiel impliqué dans une activité musculaire de l'utilisateur (P) ;
une deuxième unité de détection (103) qui détecte un angle d'une articulation de l'utilisateur (P) ;
une troisième unité de détection (104) qui détecte une charge de plante de pied de l'utilisateur (P) ;
une première unité de commande (111) qui génère un premier signal de commande pour permettre à la source d'entraînement (132) de générer une puissance conformément au signal de biopotentiel ;
une deuxième unité de commande (112) qui spécifie une phase de marche de l'utilisateur (P) sur la base de l'angle d'articulation et de la charge de plante de pied, et qui génère un deuxième signal de commande pour permettre à la source d'entraînement (132) de générer une puissance conformément à la phase ;
une unité de synthèse (114) qui synthétise le premier signal de commande et le deuxième signal de commande pour générer un signal de commande de synthèse ;
une unité de génération (131) qui génère un courant de commande sur la base du signal de commande de synthèse, et qui délivre ce courant de commande à la source d'entraînement (132) ; et
une unité de calcul qui calcule une vitesse de marche de l'utilisateur (P).

9. Système d'apprentissage de la marche (1) selon la revendication 8, dans lequel
l'unité de calcul acquiert une longueur de foulée de l'utilisateur (P) à partir d'une longueur de la jambe de l'utilisateur (P), et d'une transition de l'angle d'articulation de l'utilisateur (P) détecté par la deuxième unité de détection (103), et calcule la vitesse de marche à partir de la longueur de foulée et d'un temps de l'instant auquel le pied de l'utilisateur (P) est soulevé du sol jusqu'à l'instant auquel il est posé sur le sol.

10. Système d'apprentissage de la marche (1) selon la revendication 8 ou 9, dans lequel
le dispositif d'assistance au mouvement pouvant être porté (100) comprend :
un premier cadre (11) et un deuxième cadre (13), le premier cadre (11) et le deuxième cadre (13) étant attachés le long de la jambe de l'utilisateur (P) pour pouvoir être pliés ;
un premier manchon (21) monté sur le premier cadre (11) et placé sur une cuisse de l'utilisateur (P) ; et
un deuxième manchon (23) monté sur le deuxième cadre (13) et placé sur la jambe sous le genou de l'utilisateur (P).

11. Système d'apprentissage de la marche (1) selon la revendication 10, dans lequel
le premier manchon (21) est placé sur l'arrière de la cuisse de l'utilisateur (P), et le deuxième manchon (23) est placé sur l'avant de la jambe sous le genou de l'utilisateur (P).
